Europäisches Patentamt

European Patent Office

Office européen des brevets

(19)

(11) Veröffentlichungsnummer: **0 158 190 B1**

## (12) EUROPÄISCHE PATENTSCHRIFT

(45) Veröffentlichungstag der Patentschrift: **15.01.92**

(21) Anmeldenummer: **85103336.5**

(22) Anmeldetag: **22.03.85**

(51) Int. Cl.⁵: **G01N 33/66**, G01N 33/52, C12Q 1/00

Die Akte enthält technische Angaben, die nach dem Eingang der Anmeldung eingereicht wurden und die nicht in dieser Patentschrift enthalten sind.

(54) **Teststreifen zur Aufnahme von Blutzuckertagesprofilen und Verfahren zu seiner Herstellung sowie dessen Verwendung.**

(30) Priorität: **09.04.84 DD 261762**

(43) Veröffentlichungstag der Anmeldung:
**16.10.85 Patentblatt 85/42**

(45) Bekanntmachung des Hinweises auf die Patenterteilung:
**15.01.92 Patentblatt 92/03**

(84) Benannte Vertragsstaaten:
**AT CH DE FR GB LI SE**

(56) Entgegenhaltungen:
EP-A- 0 014 929          EP-A- 0 054 689
EP-A- 0 101 799          DD-A- 140 175
DD-A- 218 960            DE-A- 3 215 983
FR-A- 2 320 553          US-A- 4 042 335
US-A- 4 283 491

(73) Patentinhaber: **KALLIES IMPORT-EXPORT VERTRIEB GMBH**
**Götzingerstrasse 17**
**O-8360 Sebnitz(DE)**

(72) Erfinder: **Kallies, Karl-Heinz**
**Götzinger Str. 17**
**DD-8360 Sebnitz(DE)**
Erfinder: **Plaschnick, Dieter, Dipl.-Chem.**
**Thalheimer Str. 42**
**DD-4440 Wolfen(DE)**
Erfinder: **Mohr, Peter, Prof. Dr.**
**Haubachstr. 37**
**DD-1406 Hohen-Neuendorf(DE)**
Erfinder: **Thiele, Hans-Jürgen, Dr.**
**Ludwig Jahn-Str. 4**
**DD-8080 Dresden(DE)**
Erfinder: **Pahlitzsch, Conrad Johannes**
**Pestalozzistr. 45**
**DD-8360 Sebnitz(DE)**
Erfinder: **Kretschmer, Lothar, Dipl.-Chem.**
**Bergstr. 13a**
**DD-8353 Langburkersdorf(DE)**
Erfinder: **Kallies, Gert**
**Götzinger Str. 17**
**DD-8360 Sebnitz(DE)**

(74) Vertreter: **Puchberger, Rolf, Dipl. Ing. et al**
**Patentanwälte, Dipl. Ing. Georg Puchberger**
**Dipl. Ing. Rolf Puchberger Dipl. Ing. Peter**
**Puchberger Singerstrasse 13 Postfach 55**
**A-1010 Wien(AT)**

EP 0 158 190 B1

## Beschreibung

Die Erfindung betrifft einen Teststreifen nach dem Oberbegriff des Anspruches 1. Es sollen mit dem Teststreifen große oder kleine Blutzuckertagesprofile unter Alltagsbedingungen aufgenommen werden, wobei der Teststreifen sowohl vor als auch nach der Anwendung unter Normalbedingungen aufbewahrt und verschickt werden kann.

Die Erfindung betrifft ferner ein Verfahren zur Herstellung dieses Teststreifens und seine Verwendung.

Die Therapie des Diabetes ist zunehmend auf eine normoglykämische Einstellung gerichtet. Diese normoglykämische Einstellung erfordert eine Erfassung der individuellen glykämischen Zustände unter den Bedingungen des Alltags, um so Ernährungseinstellung und körperliche Aktivität zur Therapie zu überwachen und gegebenenfalls zu korrigieren. Diabetiker sterben auch heute noch zu mehr als 50% an den Spätschäden, die sich aus einer nicht ausreichend stabilen normoglykämischen Stoffwechsellage ergeben. Die Vermeidung dieser Spätschäden und womöglich eine Rückbildung bereits eingetretener Schäden zu erreichen, ist nur über eine weitgehende Senkung der Glukosewerte im Blut zu einem stabilen normoglykämischen Zustand zu realisieren.

Da die Kriterien für diese Einstellung individuell verschieden sind und von den oben genannten Faktoren mit beeinflußt werden, ist eine regelmäßige, je nach Diabetikertyp unterschiedlich häufig ärztliche Kontrolle der Schwankungen der Blutzuckerwerte im Tages- oder 24-Stunden-Verlauf eine wichtige Grundlage für die Therapie und die Verhütung von Spätfolgen.

Abhängig vom Diabetikertyp werden kleine Tagesprofile mit 5 bis 6 Bestimmungen über den Tag oder große Tagesprofile mit 11 Bestimmungen innerhalb von 24 Stunden in Abständen von einer bis zu 12 Wochen für notwendig angesehen.

Diese medizinische Forderung kann begrenzter Weise bisher

1. durch Konservierung der selbst entnommenen Blutproben und nachfolgende enzymatische Laboranalyse erfüllt werden oder

2. der Diabetiker testet seinen Blutzuckerwert mit speziellen dafür entwickelten Teststreifen, die eine halbquantitative visuelle bzw. bei Verwendung eines Reflexionsmeßgerätes eine quantitative Bestimmung ermöglichen.

Nach der 1. Methode muß der Diabetiker die Blutproben mit Kapillaren aufnehmen und jede Kapillare in ein Röhrchen mit Konservierungsflüssigkeit geben. Für diese Verfahrensweise wird ein industrielles Testbesteck gefertigt, das dann an ein Laboratorium zu schicken ist, wo die Aufbereitung und Analyse erfolgt. Die konservierten Blutproben sind ca. 10 Tage haltbar. Das Verfahren ist aufwendig und teuer und nur begrenzt anwendbar.

Mit der 2. Methode ist, wenn eine reflektometrische Auswertung erfolgt, eine quantitative Bestimmung mit ausreichender Genauigkeit gegeben. Eine Dokumentation der Ergebnisse ist jedoch wegen der Unbeständigkeit der Färbung nicht gegeben. Dieser Mangel zwingt zur sofortigen Auswertung durch den Diabetiker selbst und setzt die Anschaffung oder Bereitstellung eines Meßgerätes zur Auswertung der Teststreifen voraus.

Wegen der geringen Anzahl der Blutzuckerbestimmungen, die, von schwangeren, jugendlichen oder labilen Diabetikern abgesehen, zwischen 5 bis 11 Blutzuckerbestimmungen im Monat bzw. Quartal liegt, werden die Ausführungen der Bestimmungen aus Mangel an Routine mit einer individuellen, zusätzlichen Streuung belastet, die der Arzt nicht einschätzen kann und eine Beurteilung der Werte erschwert. Der materielle Aufwand ist unter diesen Umständen nur bei geeigneten jugendlichen oder schwangeren Diabetikern zu rechtfertigen.

Aus diesen Gründen wird eine Testung durch den Diabetiker selbst unter Alltagsbestimmungen und einer Auswertungsmöglichkeit durch den Arzt für optimal gehalten. Auf diese Weise wird es möglich, jeden Diabetiker in eine Blutzuckerprofilkontrolle unter ärztlicher Überwachung und Bewertung zu nehmen.

In Erkennung des Problems der Farbinstabilität bisher entwickelter Teststreifen gibt es Entwicklungen zur Beseitigung dieses Mangels mittels einfach oder mehrfach beschichteter Filmträgerfolien, die mit Gelatine, Enzymen, Indikatoren und Zusätzen in unterschiedlicher Weise präpariert sind.

Dem Vorteil der erreichten Farbstabilität stehen hier Mängel in der Adaptierbarkeit an Reflexionsmeßgeräte aus Gründen der Indikatorfunktion oder des Absorptionsspektrums entgegen. Außerdem wird die Reaktionsfähigkeit durch Luftfeuchtigkeit, Wärme und Licht stark beeinflußt, was den bisherigen Einsatz verhindert hat.

Die an sich bekannte Beschichtung von Filmträgerfolien mit Enzym-Indikator-Gelatine unter Zusatz von Netzmitteln, Weichmachern, Farbstoffkupplern und Härtern zeigt bei quantitativer Bestimmung der Blutzuckerwerte eine hohe Streuung, d.h. unzureichende Präzision, die z.B. durch Auswascheffekte des Indikatorsystems, welchselnde Hämatokritgehalte, unterschiedliche Nachhärtung der Schicht, durch Lagerungseinflüsse und die Geschwindigkeit der Wasseraufnahme, d.h. die Quellung, bedingt ist.

Für den praktischen Einsatz kommt es aber besonders darauf an, das Absorptionsspektrum und die Indikatorfunktion den bereits vorhandenen Meßgeräten anzupassen und eine der Anwendung entsprechende Stabilität zu erreichen.

Es ist bekannt, Enzym-Indikator-Gelatine-Schichten nachträglich mit wäßrigen Lösungen von Chrom-III-Salzen zu behandeln. Durch diese Nachbehandlung soll die Naßfestigkeit erhöht werden, indem der Vernetzungsgrad der Gelatine verändert wird. Diese Nachbehandlung ändert nur die Oberflächeneigenschaften und ist ohne Einfluß auf den funktionellen Ablauf, das Absorptionsspektrum und die Stabilität.

Es sind auch Enzym-Indikator-Gelatine-Schichten zur Glukosebestimmung bekannt, bei denen Biphenylderivate als Chromogen mit einem Fixiermittel verwendet werden, die bei der Umsetzung bei pH < 5,0 einen Semichinonimin-Farbstoff bilden, der nicht ausgewaschen wird. Die Fixierung und Stabilität ist nur dann absolut garantiert, wenn durch die zu prüfende Flüssigkeit dieser pH-Bereich nicht verschoben wird, was aber durch Blut und Seren mit einem pH-Wert um 7,0 und einer Einwirkzeit von > 30 Sekunden erfolgt. Aus diesem Grunde ist die Präzision nur in wäßrigen sauren Lösungen ausreichend und die erhaltenen Extinktionen und Färbungen mit Blut und Seren sind nicht vergleichbar.

Es ist auch bekannt, zur Verbesserung des Funktionsverlaufs zwei Enzym-Indikator-Gelatine-Schichten aufzutragen, die unterschiedliche Enzymkonzentrationen enthalten. Da auch hier, wie oben bereits geschildert, das Fixiermittel bei pH < 5,0 zur Stabilisierung eingesetzt wird, bleiben die Mängel einer hohen Streuung, Hämatokriteinfluß und Stabilität unverändert.

Aus der DD-A-140 175 ist bereits ein Teststreifen zur Bestimmung von Glukose in Körperflüssigkeiten bekannt, wobei dieser Teststreifen ein transparentes Trägermaterial, vorzugsweise aus einem unbeschichteten Filmmaterial, aufweist, auf das dann die einzelnen Testkomponenten und der Indikatorfarbstoff sowie Gelatine und ein Stabilisator aufgebracht werden. Ein weiterer Teststreifen ist aus der US-A-4 283 491 bekannt geworden. Hier werden analytische Elemente mit verbesserter Reagenzienstabilität beschrieben, wobei der Teststreifen einen ganz speziellen Aufbau und eine ganz spezielle Zusammensetzung besitzt.

Die beschriebenen Verfahren und Teststreifen bzw. beschichteten Folien weisen alle den Nachteil auf, daß sie nicht an die seit 10 Jahren im Handel befindlichen Reflexionsmeßgeräte angepaßt werden können. Für den Einsatz zur Bestimmung von Glukosetagesprofilen sind sie ebenso wie die bisher im Handel befindlichen Teststreifen nur bedingt geeignet.

Das erfindungsgemäße Ziel ist ein Teststreifen, der vom Diabetiker mit Blut betropft wird, das nach zeitlich begrenzter Einwirkung abgewischt und danach dem Arzt zur Kontrolle und Auswertung zugeschickt wird.

Der Teststreifen soll so gestaltet sein, daß Uhrzeit, Datum und Name auf jedem Teststreifen notiert werden können. Die Stabilität, die angestrebt wird, soll den Postversand ermöglichen und eine sofortige oder beliebig spätere genaue Glukosebestimmung garantieren.

Das Verfahren zur Herstellung des Teststreifens ist darauf gerichtet, die Funktion der Filmreaktionsfolie so zu stabilisieren, daß sie unter Normalbedingungen wenigstens 4 Wochen konstant erhalten bleibt und nach Benutzung die eingetretene Farbreaktion nicht nur stabil bis zur Auswertung innerhalb von 4 Wochen, sondern darüberhinaus als Beleg stabil bleibt, ja sogar als Farbvergleich für visuelle Bewertungen bei einzelnen, zwischenzeitlich eventuell angeordneten Bestimmungen eingesetzt werden kann.

Es ist weiter das Ziel, durch die verfahrensmäßige Bearbeitung die Funktion zwischen Glukosekonzentration und Extinktion sowie das Absorptionsspektrum so einstellen zu können, daß eine Adaption an vorhandene bzw. handelsübliche Meßgeräte in unkomplizierter Weise gelingt.

Es ist das Ziel, für die bekannte enzymatische Indikatorreaktion mit Glukose eine einfache Verfahrensweise ohne komplizierten mehrschichtigen Aufbau, ohne enzymbedingte, unterschiedliche Reaktionsführung oder extrem niedrige Schichtdicke zu finden, die in umfassender Weise die Zielstellung erfüllt.

Es ist Aufgabe der Erfindung, die für die Blutzuckertagesprofile notwendigen Voraussetzungen und Forderungen nach Stabilität der Teststreifen, Stabilität der Färbung, Präzision der Ergebnisse, Adaptierbarkeit an handelsüblichen Reflexionsmeßgeräte und Einstellbarkeit auf unterschiedliche Meßbereiche ohne komplizierte, mehrschichtige Reaktionsführung oder besonders dünne Beschichtung und sich daraus ableitende Nachteile der Funktion bzw. des Absorptionsspektrums zu erreichen.

Es ist also Aufgabe der Erfindung, durch ein einfaches, technisch unkompliziertes Verfahren der Nachbehandlung die Funktion der in bekannter Weise hergestellten Enzym-Indikator-Gelatine-Schichten so zu verändern, daß das Wasseraufnahmevermögen in 1 Minute sich um 10 bis 25% erhöht, eine visuell andere Farbreaktion eintritt, statt des leuchten blauen, sauer fixierten Farbstoffes eine graue bis blaugraue Färbung entsteht, die aber ebenfalls bei 610 bis 625 nm gemessen werden kann, gleichzeitig die Präzision erhöht, die Hämatokritabhängigkeit vermindert und eine Stabilität erreicht wird, die sowohl den Versand der Teststreifen wie auch der Profilwerte gestattet.

Zur Lösung dieser Aufgabe wird eine ca. 20 $\mu$ starke Enzym-Indikator-Gelatine-Schicht, deren Zusammensetzung dem seit über 20 Jahren bekannten Enzym-Indikator-Verhältnis entspricht und die für eine Gelatineschicht aus der Fotoindustrie bekannten Weichmacher, Netzmittel, Vernetzungsmittel oder Farbstoffkuppler enthalten kann, auf eine Trägerfolie aufgetragen und in bekannter Weise getrocknet.

Die getrocknete, beschichtete Filmfolie wird 5 bis 30 Sekunden, vorzugsweise 5 bis 10 Sekunden, durch eine 0,01 bis 0,1 molar vorliegenden Komplexbildner, vorzugsweise Komplexon®I (Nitrilotriessigsäure) oder Komplexon®III (Ethylendiamintetraessigsäure-Dinatriumsalz), und anschließend 60 bis 120 Sekunden durch eine acyclische Hydroxiamine bzw. Aminoderivate enthaltende 0,01 bis 1 molare, vorzugsweise 0,02 bis 0,5 molare Lösung geführt. Als acyclische Hydroxiamine sind beispielsweise Cholin oder Aminotrishydroxi-methyl-methan geeignet, als Aminoderivate Collidin, Chinolin oder Piperazin. Komplexon® ist ein Warenzeichen der Firma Uetikon, Schweiz.

Es ist aber auch möglich, die beschichtete Filmfolie nicht nacheinander sondern in einer Lösung zu behandeln. Die derartig nachbehandelte Schicht erfüllt die Forderungen, die an die Aufnahmemittel für Blutzuckertagesprofile gestellt werden und ist an alle handelsüblichen Reflexionsmeßgeräte adaptierbar.

Als Filmfolie für die aufzubringende Reaktionsschicht werden wasserunlösliche Kunststoffolien verwendet, die farblos oder auch eingefärbt sein können.
Die Beschichtung der Filmfolien kann ganzflächig oder auch nur teilweise in beliebiger, technisch möglicher Breite vorgenommen werden, je nach Anwendungszweck oder ökonomischer Forderung.

Für die Auswertung der beschichteten Filmfolien auf Reflexionsfotometern wird als Träger zweckmäßigerweise eine weiße Folie verwendet. Eine universelle Lösung wird erhalten, wenn eine durchsichtige, beschichtete Filmfolie mit einem weißen Trägerkarton durch Versiegeln oder Verkleben teilweise verbunden wird, so daß damit sowohl die Reflexions- als auch die Durchlichtmessung am gleichen Teststreifen möglich ist. Zum Verkleben können als Klebebänder mit Haftkleber beschichtete PVC-, Polyester-, Celluloseacetat- o.ä. Folien eingesetzt werden. So hergestellte Teststreifen können Druckvermerke für Namen, Datum und Uhrzeit enthalten, sind damit beschriftbar und nicht verwechselbar.

Der nach dem erfindungsgemäßen Verfahren hergestellte Teststreifen liefert farbstabile, dokumentationsfähige, dem jeweiligen Glukosegehalte entsprechende Färbungen, die zu beliebiger Zeit durch den Diabetiker selbst, den betreuenden Arzt oder ein Labor auswertbar sind. Der Teststreifen ist verfahrensgemäß so hergestellt, daß er bei Zimmertemperatur ausreichen haltbar ist und den Postversand vom Arzt zum Diabetiker und nach Benutzung vom Diabetiker zum Arzt ohne Verschlechterung der Ergebnisse verträgt.

Der erfindungsgemäß hergestellte Teststreifen ist im Durchlicht auswertbar und kann außerdem auf Reflexionsmeßgeräte eingestellt werden.

Es ist möglich, einen Meßbereich, vorzugsweise für jugendliche und schwangere Diabetiker, einzustellen, der hauptsächlich dem hypoglykämischen bis zum schwach hyperglykämischen Bereicht entspricht, oder den Meßbereich mit dem Schwergewicht auf den normo- bis hyperglykämischen zu dehnen.
Der Teststreifen löst damit die Forderungen der Therapiekontrolle für alle Diabetesformen und einer universellen Auswertung und Anwendung.

Ausführungsbeispiele

Beispiel 1

Eine 7%ige Gelatinelösung, die in 100 ml 2000 U POD und 8000 U GOD, 0,3 g o-Tolidin sowie 0,45 g eines Naphtholderivates als Kuppler und zusätzlich Netzmittel und Weichmacher enthält, wird vor der Beschichtung mit einem Härter, z.B. einem Chrom-III-Salz versetzt. Mit dieser Lösung wird eine ca. 20 μ starke Schicht auf eine Filmfolie aus Polyester aufgetragen und getrocknet.
Im Tauchverfahren wird die Schicht 10 Sekunden mit einer 0,01 molaren Komplexon®-III-Lösung (Ethylendiamintetraessigsäure-Dinariumsalz) angequollen, überschüssige Flüssigkeit entfernt und sofort anschließend die Filmbahn durch ein zweites Tauchbad geführt, das 0,05 mol Collidin enthält und in dem die Schicht 90 Sekunden weiter gequollen bzw. nachbehandelt wird. Anschließend wird die gequollene, nachbehandelte Schicht in bekannter Weise getrocknet.

Beispiel 2

In anderen Ausführungen gemäß Beispiel 1 wird für die Nachbehandlung in dieser Weise anstelle von Collidin
- Piperazin in einer 0,03 molaren Lösung,
- Cholin in einer 0,04 molaren Lösung,
- Amino-trishydroxymethyl-methan in einer 0,02 molaren Lösung eingesetzt.

Beispiel 3

In einer anderen Ausführung, analog Beispiel 1, wird die Quellung und Nachbehandlung mit einer Lösung durchgeführt. Dazu werden 0,01 Mol Komplexon®-III (Ethylendiamintetraessigsäure-Dinatriumsalz) und 0,05 Mol Amino-trishydroxymethyl-methan zusammen in 1000 ml Wasser gelöst und die Filmreaktionsschicht in dieser Lösung 80 Sekunden gequollen bzw. nachbehandelt.

Beispiel 4

In einer anderen Ausführung, gemäß Beispiel 1, wird der Nachbehandlungslösung 0,01 % Pinaverdol zugesetzt, wodurch sich die Reaktionsschicht schwach rotviolett färbt, ohne daß dadurch die späteren Messungen und Meßergebnisse gestört werden.

Beispiel 5

Es wird gemäß Beispiel 1 gearbeitet. Die beschichtete Filmfolie wird mit der Reaktionsschicht nach oben und der Filmfolie zum Träger hin auf einen weißen Trägerkarton aufgesiegelt.

Beispiel 6

Man verfährt wie in dem Beispiel 1. Die beschichtete Filmfolie wird mit der Reaktionsschicht zum Träger hin auf weißes Filterpapier mittels Klebefolie beidendig aufgeklebt, wobei die Klebefolie die beschichtete Filmfolie völlig und den Träger jeweils nur zu 5 mm überdeckt.

Beispiel 7

Man arbeitet gemäß Beispiel 1. Als Träger wird mit Gelatine gestrichener Kunstdruckkarton eingesetzt. Es wird eine auf der Rückseite mit Gelatine substrierte Filmreaktionsfolie verwendet. Die Verbindung zwischen Filmreaktionsfolie und Trägerkarton erfolgt anschließend einendig mit einem wäßrigen Klebemittel.

**Patentansprüche**

1. Teststreifen zur Aufnahme von Blutzuckertagesprofilen, bestehend aus einem Träger und einer Filmreaktionsfolie, auf der eine GOD, POD sowie o-Tolidin enthaltende Enzym-Indikator-Gelatine-Schicht, gegebenenfalls unter Zusatz von Weichmachern, Netzmitteln, Vernetzungsmitteln und/oder Farbstoffkupplern, aufgebracht ist, gekennzeichnet durch eine mittels Komplexbildner und acyclische Hydroxyamine bzw. Aminoderivate nachbehandelte beschichtete Filmreaktionsfolie, die mit dem Träger verbunden ist.

2. Teststreifen nach Anspruch 1, dadurch gekennzeichnet, daß die Filmfolie aus einer wasserunlöslichen Kunststoffolie besteht.

3. Teststreifen nach den Ansprüchen 1 und 2, dadurch gekennzeichnet, daß die Kunststoffolie farblos, weiß oder farbig ist.

4. Teststreifen nach den Ansprüchen 1 bis 3, dadurch gekennzeichnet, daß die Filmfolie ganzflächig oder teilweise mit der Reaktionsschicht überzogen ist.

5. Teststreifen nach Anspruch 1, dadurch gekennzeichnet, daß der Träger aus weißer Kunststoffolie besteht.

6. Teststreifen nach Anspruch 1, dadurch gekennzeichnet, daß der Träger aus weißem Trägerkarton besteht.

7. Teststreifen nach den Ansprüchen 1 bis 6, dadurch gekennzeichnet, daß die Filmreaktionsfolie mit dem Träger durch Versiegeln oder Verkleben verbunden ist.

8. Teststreifen nach den Ansprüchen 1 bis 7, dadurch gekennzeichnet, daß die Filmreaktionsfolie mit dem Träger teilweise verbunden ist.

9. Teststreifen nach den Ansprüchen 1 bis 8, dadurch gekennzeichnet, daß die Filmreaktionsfolie mit dem Träger durch mit Haftkleber beschichtete, die Meßfunktion nicht beeinträchtigende PVC-, Polyester- oder Celluloseacetatfolie verbunden ist.

10. Verfahren zur Herstellung eines Teststreifens nach den Ansprüchen 1 bis 9, dadurch gekennzeichnet, daß die beschichtete Filmreaktionsfolie mit Komplexbildnern und acyclischen Hydroxyaminen bzw. Aminoderivaten gequollen und nachbehandelt und danach mit einem Träger verbunden wird.

11. Verfahren nach Anspruch 10, dadurch gekennzeichnet, daß der Komplexbildner in einer 0,01 bis 0,1 molaren Konzentration angewendet wird.

12. Verfahren nach den Ansprüchen 10 und 11, dadurch gekennzeichnet, daß man den Komplexbildner 5 bis 30 Sekunden, vorzugsweise 5 bis 10 Sekunden,einwirken läßt.

13. Verfahren nach den Ansprüchen 10 bis 12, dadurch gekennzeichnet, daß als Komplexbildner Komplexon® I (Nitrilotriessigsäure) eingesetzt wird.

14. Verfahren nach den Ansprüchen 10 bis 13, dadurch gekennzeichnet, daß als Komplexbildner Komplexon® III (Ethylendiamintetraessigsäure-Dinatriumsalz) eingesetzt wird.

15. Verfahren nach Anspruch 10, dadurch gekennzeichnet, daß die acyclischen Hydroxyamine bzw. Aminoderivate in einer 0,01 bis 1 molaren, vorzugsweise 0,02 bis 0,5 molaren Konzentration angewendet werden.

16. Verfahren nach den Ansprüchen 10 und 15, dadurch gekennzeichnet, daß man die acyclischen Hydroxyamine bzw. Aminoderivate 60 bis 120 Sekunden einwirken läßt.

17. Verfahren nach den Ansprüchen 10, 15 und 16, dadurch gekennzeichnet, daß als acyclische Hydroxyaminoverbindung Cholin eingesetzt wird.

18. Verfahren nach den Ansprüchen 10, 15 und 16, dadurch gekennzeichnet, daß als acyclische Hydroxyaminoverbindung Amino-trishydroxy-methyl-methan eingesetzt wird.

19. Verfahren nach den Ansprüchen 10, 15 und 16, dadurch gekennzeichnet, daß als Aminoderivat Collidin, Piperazin oder Chinolin eingesetzt wird.

20. Verfahren nach den Ansprüchen 10 bis 19, dadurch gekennzeichnet, daß die Filmreaktionsfolie mit Komplexbildnern und acyclischen Hydroxyaminen bzw. Aminoderivaten zugleich behandelt wird.

## Claims

1. Test strip for determining blood sugar day profiles, consisting of a carrier and a film reaction foil, to which an enzyme indicating gelatine layer is applied, containing GOD, POD as well as o-tolidine, if required by addition of plasticisers, wetting agents, cross-linking agents and/or dye-stoff couplers, characterized by a coated film reaction foil, being aftertreated by complex forming substances and acylic hydroxyamines or amino derivates, which foil is connected to the carrier.

2. Test strip according to claim 1, characterized in that, the film foil consists of a water insoluble plastic foil.

3. Test strip according to the claims 1 and 2, characterized in that, the plastic foil is colourless, white or coloured.

4. Test strip according to the claims 1 to 3, characterized in that, the film foil is coated with a reagent layer on its entire face or a part thereof.

5. Test strip according to claim 1, characterized in that, the carrier consists of a white plastic foil.

6. Test strip according to claim 1, characterized in that, the carrier consists of a white carrier board.

7. Test strip according to the claims 1 to 6, characterized in that, the film reaction foil is affixed to the carrier by sealing or glucing.

8. Test strip according to the claims 1 to 7, characterized in that, the film reaction foil is partially affixed to the carrier.

9. Test strip according to the claims 1 to 8, characterized in that, the film reaction foil is affixed to the carrier by polyvinyl chloride, polyester or cellulose acetate foil, being coated by an adhesive not impairing the function of measuring.

10. Process for the manufacture of a test strip according to the claims 1 to 9, characterized in that the coated film reaction foil is swollen and aftertreated with complex forming substances and acyclic hydroxyamines or amino derivates and is then affixed to the carrier.

11. Process for the manufacture of a test strip according to claim 10, characterized in that the complex forming substances are utilised in a molar concentration of 0,01 to 0,1.

12. Process for the manufacture of a test strip according to the claims 10 and 11, characterized in that the complex forming substances are allowed to react 5 to 30 seconds, preferably 5 to 10 seconds.

13. Process according to the claims 10 to 12, characterized in that as a complex forming substance Komplexon[R]I (nitrilotriacetic acid) is used.

14. Process according to the claims 10 to 13, characterized in that as complex forming substance Komplexon[R]III (ethylene diamino tetraacetic acid-disodium salt) is used.

15. Process according to claim 10, characterized in that the acylic hydroxyamines or amino derivates are utilized in a molar concentration of 0,01 to 1, preferably in a molar concentration of 0,02 to 0,5.

16. Process according to the claims 10 and 15, characterized in that the acyclic hydroxyamines or amino derivates are allowed to react 60 to 120 seconds.

17. Process according to the claims 10, 15 and 16, characterized in that as acyclic hydroxyamine composition choline is used.

18. Process according to the claims 10, 15 and 16, characterized in that as acyclic hydroxyamine compound amino-tri-hydroxy-methyl-methane is utilized.

19. Process according to the claims 10, 15 and 16, characterized in that as amino derivate collidine, piperazine oder chinoline is utilized.

20. Process according to the claims 10 to 19, characterized in that the film reaction foil is treated simultaneously by the complex forming substances and acyclic hydroxy amines or amino derivates.

**Revendications**

1. Bande de test pour l'enregistrement des profiles quotidiens de glucose de sang, composée d'un porteur et d'une feuille film de réaction, étant couverte d'une couche gélatineuse enzyme-indicateur renfermant GOD, POD et o-tolidine, le cas échéant par addition des plastifiants, agents mouillants, agents de réticulation et/ou couplants pour matières colorantes, caractérisée par une feuille film de réaction couverte, revêtue et traitée postérieurement par des complexants et des hydroxyamines acycliques et/ou dérivés d'amino, qui est liée avec le porteur.

2. Bande de test selon la révendication 1, caractérisée en ce que la feuille film est composée par une feuille en matière plastique insoluble dans l'eau.

3. Bande de test selon les révevendications 1 et 2, caractérisée en ce que la feuille an matière plastique est incolorée, blanche ou colorée.

4. Bande de test selon les révevendications 1 à 3, charactérisée en ce que la feuille film est couverte complètement ou partiellement par la couche de réaction.

5. Bande de test selon la révevendication 1, charactérisée en ce que le porteur est composé par une feuille en matière plastique blanche.

6. Bande de test selon la révevendication 1, charactérisée en ce que le porteur est composé par un carton porteur blanc.

7. Bande de test selon les révevendications 1 à 6, charactérisée en ce que la feuille film de réaction est liée par scellement ou par collage au porteur.

8. Bande de test selon les révevendications 1 à 7, charactérisée en ce que la feuille film de réaction est partiellement liée au porteur.

9. Bande de test selon les révevendications 1 à 8, charactérisée en ce que la feuille film de réaction est liée au porteur par une feuille PVC, polyester ou acetate de cellulose, étant couverte par un adhésive non-influençant la fonction de mesure.

10. Procès pour la manufacture d'une bande de test selon les révendications 1 à 9, charactérisé en ce que la feuille film de réaction couverte est gonflée et traitée postérieurement avec complexants et hydroxyamines acycliques et/ou dérivés d'amino et est après liée au porteur.

11. Procès pour la manufacture d'une bande de test selon la révendication 10, charactérisé en ce que l'on utilise le complexant en concentration molaire de 0,01 à 0,1.

12. Procès pour la manufacture d'une bande de test selon les révendications 10 et 11, charactérisé en ce que l'on fait agir le complexant 5 à 30 secondes, préfèrablement 5 à 10 secondes.

13. Procès selon les révendications 10 à 12, charactérisé en ce que l'on utilise comme complexant Komplexon$^R$I (acide nitrilotriacetique).

14. Procès selon les révendications 10 à 13, charactérisé en ce que l'on utilise comme complexant Komplexon$^R$III (acide ethylenediaminetetraacetique-disodium sel).

15. Procès selon la révendication 10, charactérisé en ce que l'on utilise les hydroxyamines acycliques ou dérivés d'amino à une concentration molaire de 0,01 à 1, préfèrablement 0,02 à 0,5.

16. Procès selon les révendications 10 à 15, charactérisé en ce que l'on fait agir les hydroxyamines acycliques ou dérivés d'amino 60 à 120 secondes.

17. Procès selon les révendications 10, 15 et 16, charactérisé en ce que l'on utilise comme composé hydroxyamine acyclique choline.

18. Procès selon les révendications 10, 15 et 16, charactérisé en ce que l'on utilise comme composé hydroxyamine acyclique aminotrihydroxymethylmethane.

19. Procès selon les révendications 10, 15 et 16, charactérisé en ce que l'on utilise comme dérivé d'amino collidine, piperazine ou chinoline.

20. Procès selon les révendications 10 à 19, charactérisé en ce que la feuille film de réaction est traitée simultanément avec les complexants et les hydroxyamines acycliques et/ou dérivés d'amino.